# EUROPÄISCHE PATENTSCHRIFT

(11) **EP 2 101 659 B1**
(45) Veröffentlichungstag und Bekanntmachung des Hinweises auf die Patenterteilung: **04.04.2012**
(21) Anmeldenummer: 07856514.0
(22) Anmeldetag: 10.12.2007
(51) Int. Cl.: A61B 17/34, A61B 17/32, A61B 17/30, A61B 17/00

(54) **VORRICHTUNG ZUR FIXIERUNG UND/ODER MANIPULATION VON GEWEBE**
DEVICE FOR FIXING AND/OR MANIPULATING TISSUE
DISPOSITIF POUR IMMOBILISER ET/OU MANIPULER UN TISSU

(30) Priorität: 10.12.2006 DE 102006058447
(43) Veröffentlichungstag der Anmeldung: 23.09.2009
(73) Patentinhaber: Philipps-Universität Marburg, 35037 Marburg (DE)
(72) Erfinder: MAISCH, Bernhard, 35043 Marburg (DE); RUPP, Heinz, 35043 Marburg (DE); RUPP, Thomas Philipp, 35039 Marburg (DE); SCHÜLER, Ekkehard, 35041 Marburg (DE); RUPP, Karin, 35043 Marburg (DE)
(74) Vertreter: Buchhold, Jürgen
(86) Internationale Anmeldenummer: PCT/EP2007/010740
(87) Internationale Veröffentlichungsnummer: WO 2008/071367

(56) Entgegenhaltungen:
- WO-A-96/40368
- WO-A-99/13785
- WO-A-99/13936
- WO-A-2005/016157

## Beschreibung

Die Erfindung betrifft eine Vorrichtung zur Fixierung und/oder Manipulation von Gewebe gemäß dem Oberbegriff von Anspruch 1.

Vorrichtungen zur Fixierung und/oder Manipulation von Gewebe sind bereits bekannt. So beschreibt beispielsweise US 5 827 216 A (= WO 96/40368 A1)eine Vorrichtung zum Fixieren, Anheben und Punktieren des Perikards, mit einem das Gewebe des Perikards und/oder die darunter liegenden Bereiche penetrierenden Körper gemäß dem Oberbegriff des Anspruchs 1. Hierbei handelt es sich vorzugsweise um eine Hohlnadel, die längsverschieblich in einem Führungskörper gelagert und in einen Innenraum eines Ansaugkopfes für das Gewebe einbringbar ist. Der Ansaugkopf ist am vorderen (distalen) Ende des Führungskörpers ausgebildet und mit einer seitlichen Öffnung versehen, durch die der Innenraum von außen zugänglich ist. Durch den Führungskörper hindurch kann in dem Innenraum ein Unterdruck ausgebildet werden, indem eine Saugeinrichtung an die Vorrichtung angeschlossen wird. Legt man den Ansaugkopf mit der seitlichen Öffnung auf das Perikard auf, wird dieses durch die Öffnung hindurch in den Innenraum eingesaugt. Es bildet sich eine Blase, die anschließend mit der Nadel punktiert werden kann. Durch die Hohlnadel hindurch können Flüssigkeiten abgesaugt oder Medikamente zugeführt werden. Man kann über die Hohlnadel auch einen Draht in den Herzbeutel einführen.

Untersuchungen an verschiedenen Geweben haben gezeigt, dass eine ausreichende Fixierung des Perikards und ein Zugang zum Herzbeutel nur dann erfolgreich waren, wenn das eingesaugte Gewebe hinreichend dünn war. Ist das Gewebe hingegen verdickt, beispielsweise als Folge von Verfettungen, Entzündungen oder Vernarbungen, so wird der Innenraum des Ansaugkopfes nur mit Gewebe ausgefüllt. Es bildet sich keine Blase, die einen Zugang zum Herzbeutel ermöglicht. Die im Führungskörper geführte Nadel sticht nur in das den Innenraum ausfüllende Gewebe ein und gelangt nicht in den darunter liegenden Bereich des Herzbeutels. Ein verdicktes Perikard kann ferner die Öffnung soweit verstopfen, dass nur wenig oder gar kein Gewebe in den Innenraum gelangt. Eine zuverlässige Fixierung des Gewebes ist damit unmöglich, ebenso eine Punktion mit der Nadel oder einem anderen penetrierenden Körper.

Ein weiterer Nachteil der genannten Vorrichtungen besteht oft darin, dass das Gewebe innerhalb des Ansaugkopfes nicht ausreichend gespannt werden kann. In diesem Fall würde das Perikard beim Versuch es zu durchstechen ausweichen. Ein penetrierender Körper, z.B. ein flexibler, meist stumpfer Draht, würde sich abwinkeln und nicht das Perikard(zelt) perforieren.

US 5 972 013 A (= WO 99/13785 A1) offenbart eine Vorrichtung, die ebenfalls einen minimal-invasiven Zugriff zum Perikard des Herzens eines Menschen oder eines Tieres ermöglichen soll. Die Nadel ist in einem Lenkrohr geführt, das an seinem distalen Ende mit einer stirnseitigen Öffnung zum Ansaugen des Perikards versehen ist. Führt man die Nadel nach dem Ansaugen des Gewebes in den Innenraum ein, wird das Nadelende mittels einer Lenkvorrichtung in einem Winkel quer zur Bewegungsrichtung umgelenkt, so dass die Nadel schräg in das Perikard eindringt.

Von Nachteil ist auch hier, dass verdicktes Gewebe nicht oder nur unzureichend in den Innenraum des Ansaugkopfes eingesaugt werden kann, so dass die Punktion mit dem penetrierenden Körper nicht oder nur im Gewebe selbst erfolgen kann. Problematisch ist ferner, dass die Manipulation mit der Nadel im Wesentlichen senkrecht zur Oberfläche des Gewebes und damit senkrecht zu dem darunter liegenden, meist schlagenden Herzen durchgeführt wird. Eine versehentliche Punktion des Herzmuskels ist jedoch eine lebensbedrohliche Verletzung, welche in sehr kurzer Zeit sogar zum Tod des Patienten führen kann.

WO 99/13936 A1 offenbart ebenfalls eine Vorrichtung und ein Verfahren für den minimal-invasiven Zugriff auf das Perikard eines menschlichen oder tierischen Patienten. Die dargestellte Vorrichtung hat einen Führungskörper, in dessen Hohlraum eine Hohlnadel längsverschieblich geführt ist. Das distale Ende des Führungskörpers weist eine starre Schulter auf, um das mittels eines Vakuums stirnseitig in den Führungskörper eingesaugte Perikardgewebe zu stützen.

WO 2005/016157 A beschreibt eine weitere Vorrichtung zum Punktieren oder einer anderen Manipulation des menschlichen oder tierischen Gewebes, wobei die Anheftung des Gewebes mittels einer Anheftungserfassungsvorrichtung detektiert und mittels einer Anzeigevorrichtung zuverlässig erkannt und signalisiert wird. Eine solche Vorrichtung ermöglicht den Zugang in den Herzbeutel nach Durchstechen des Perikards. Sie kann für jedes menschliche oder tierische Gewebe bzw. Organ eingesetzt werden, vor allem im Rahmen der minimal-invasiven Chirurgie, wo es keine direkte Sichtkontrolle gibt und wo sichergestellt werden muss, dass ein Gewebe bzw. Organ für eine Manipulation an eine spezielle Anordnung, z. B. an eine Vorrichtung zum Punktieren, angeheftet ist.

Ziel der Erfindung ist es, diese und weitere Nachteile des Standes der Technik zu überwinden und eine Vorrichtung zu schaffen, die eine stets zuverlässige Fixierung und/oder Manipulation eines Gewebes ermöglicht, selbst wenn dieses verdickt ausgebildet sein sollte. Ein in der Vorrichtung geführter penetrierender Körper soll das Gewebe rasch und präzise durchdringen und einen unter dem Gewebe liegenden Bereich optimal erreichen. Die Vorrichtung soll ferner einfach und kostengünstig aufgebaut und leicht zu handhaben sein.

Hauptmerkmale der Erfindung sind in Anspruch 1 angegeben. Ausgestaltungen sind Gegenstand der Ansprüche 2 bis 12.

Bei einer Vorrichtung zur Fixierung und/oder Manipulation von Gewebe und/oder darunter liegender Bereiche, mit wenigstens einem das Gewebe und/oder die darunter liegenden Bereiche penetrierenden Körper und mit einem Führungskörper für den penetrierenden Körper, wobei der Führungskörper Mittel zur Führung des penetrierenden Körpers aufweist, wobei der Führungskörper endseitig einen Ansaugkopf für das Gewebe hat oder bildet, wobei der Führungskörper innerhalb des Ansaugkopfes einen Innenraum aufweist, mit wenigstens einer im seitlichen Bereich des Führungskörpers ausgebildeten Öffnung zur Aufnahme des zu punktierenden und/oder zu manipulierenden Gewebes, und wobei der Führungskörper Mittel aufweist, zum Einbringen eines Vakuums in den Innenraum, sieht die Erfindung vor, dass im Bereich der Öffnung des Ansaugkopfes wenigstens ein Mittel zur mechanischen Fixierung und/oder Festlegung des Gewebes am Ansaugkopf vorgesehen ist, wobei das wenigstens eine Mittel zur mechanischen Fixierung und/oder Festlegung des Gewebes am Ansaugkopf ein elastisches Spann- oder Klemmelement ist.

Durch die Mittel zur mechanischen Fixierung und/oder Festlegung des Gewebes am Ansaugkopf ist es gelungen, eine gegenüber herkömmlichen Vorrichtungen deutlich verstärkte und zuverlässigere Anheftung eines Gewebes zu erreichen. Dieses wird nicht nur von dem Ansaugkopf angesaugt, sondern zusätzlich mechanisch fixiert, was eine deutlich verbesserte Handhabung der gesamten Vorrichtung, insbesondere des penetrierenden Körpers ermöglicht. Die erfindungsgemäße Vorrichtung kann dabei für jedes ansaugbare Gewebe, insbesondere für menschliches oder tierisches Gewebe beliebiger Dicke eingesetzt werden, vor allem im Rahmen der minimal-invasiven Chirurgie, wo es keine direkte Sichtkontrolle gibt und wo sichergestellt werden muss, dass ein Gewebe für eine spätere Manipulation an eine spezielle Anordnung, z.B. an eine Vorrichtung zum Punktieren und/oder zur Zu- oder Abführung von Materialien in und/oder aus dem Bereich unterhalb des Gewebes, angeheftet ist.

Die erfindungsgemäße Lösung eröffnet dabei völlig neue Handhabungsmöglichkeiten der Vorrichtung, insbesondere oder gerade beim Anheben eines ggf. verdickten Perikards, denn bei der neuen Anordnung erfolgt eine zusätzliche mechanische Fixierung des Gewebes am Ansaugkopf, gefolgt von einer Manipulation, z.B. dem Vorschieben eines Drahtes, eines Dilatators oder einer Nadel, vorzugsweise senkrecht zur Klemmung in das Gewebe mit variabler Dicke. Durch die Klemmung kann das Gewebe der Manipulation nicht mehr ausweichen und selbst ein flexibler Draht bewirkt eine stets zuverlässige und präzise Perforation des Gewebes. Im Falle eines flexiblen Drahtes, kann eine weitere Führung für den Draht in der Anordnung vorhanden sein, so dass er der Gegenkraft beim Einstechen in das Gewebe nicht ausweichen kann. Der Vorteil dieser Anordnung mit flexiblem Draht ist, dass nach Durchstechen des Perikards der flexible Draht im Herzbeutel nicht mehr geführt ist und daher nicht das dem Perikard gegenüberliegende Epikard verletzen kann. Vielmehr wird der Führungsdraht sich im Herzbeutel frei fortbewegen. Nach Zurückziehen des Instrumentes kann der Führungsdraht im Herzbeutel verbleiben und wird z.B. zum Vorschieben eines Dilatators verwendet.

Die erfindungsgemäße Lösung sieht weiter vor, dass das wenigstens eine Mittel zur mechanischen Fixierung und/oder Festlegung des Gewebes am Ansaugkopf elastische Spann- oder Klemmelemente sind. Diese sorgen dafür, dass das Gewebe einen stets zuverlässigen Halt am Ansaugkopf findet. Jedes elastische Spann- oder Klemmelement kann dabei eine elastische Lippe, Feder oder Zunge bilden, die zumindest teilweise in die Öffnung hineinragt. Alternativ kann das elastische Spann- oder Klemmelement einen umlaufenden elastischen Rand für die Öffnung bilden.

Durch diese Ausgestaltung werden die elastischen Spann- oder Klemmelemente zunächst von dem Gewebe beim Einsaugen in den Innenraum des Ansaugkopfes zunächst auseinander gedrückt. Sie weichen z.B. in den Innenraum aus und verhaken sich mit dem Gewebe, sobald die Saugkraft nachlässt und eine äußere Kraft auf das Gewebe einwirkt.

Dabei ist es von Vorteil, wenn der von den elastischen Spann- oder Klemmelementen begrenzte Öffnungsquerschnitt kleiner ist als der Querschnitt des von der Öffnung begrenzten Innenraums. Beim Einsaugen des Gewebes vergrößert sich mithin der von den elastischen Spann- oder Klemmelementen begrenzte Öffnungsquerschnitt. Zieht man anschließend an dem Gewebe, wird die Öffnung verkleinert. Das Gewebe wird am Ansaugkopf fixiert; die Spann- oder Klemmelemente erzeugen eine Rückhaltekraft, die das Gewebe zusätzlich am Ansaugkopf fixiert. Dieser ermöglicht eine sehr viel bessere Gewebefixierung. Die Vorrichtung kann beispielsweise zum Anheben von relativ dickem Gewebe gedreht werden, um so z.B. ein äußeres oder oberes Gewebe von einem inneren Gewebe oder Bereich abzuheben.

Die elastischen Spann- oder Klemmelemente können aktiv beweglich oder veränderbar sein, wobei man einen mechanischen, elektrischen, magnetischen, hydraulischen oder pneumatischen Antrieb verwenden kann.

Zweckmäßig ist der penetrierende Körper außerhalb des Ansaugkopfes gleitgeführt. Dadurch erfolgt auch die Manipulation selbst außerhalb des Ansaugkopfes. Am Beispiel des Zugangs in das Perikard lässt sich der Ansaugkopf nach erfolgter Gewebeanheftung um ca. 90 Grad nach rechts drehen, wobei das angeheftete Gewebe nach oben gezogen wird. Nach der Drehung wird der penetrierende Körper (z.B. eine Nadel) in die entstandene Gewebefalte oder das entstandene "Zelt" eingeschoben; der darunter liegende Raum lässt sich optimal erreichen. Ein solches Vorgehen kann vermutlich bei einer Vielzahl an Patienten durchgeführt werden, deren Perikarddicke 2 bis 3 mm beträgt.

Konstruktiv ist es günstig, wenn die Mittel zur Führung des penetrierenden Körpers getrennt von den Mitteln zum Einbringen eines Vakuums in den Innenraum ausgebildet sind. Dies vereinfacht nicht nur den Aufbau der Vorrichtung. Der penetrierende Körper muss vor allem nicht mehr gegenüber dem Vakuum abgedichtet werden.

Der penetrierende Körper kann - je nach Anwendungszweck - eine Nadel, eine Elektrode, ein Draht, eine Schlinge, eine Schneidvorrichtung o.dgl. sein.

Eine wichtige Weiterbildung der Erfindung sieht vor, dass das Mittel zum Einbringen eines Vakuums in den Innenraum ein Vakuumkanal ist, der mit einer Vakuumöffnung im Innenraum mündet, wobei die Vakuumöffnung in einem Abstand vom Rand der Öffnung ausgebildet ist. Dadurch wird die Vakuumöffnung beim Einsaugen des Gewebes in den Innenraum des Ansaugkopfes nicht so schnell verdeckt. Es kann deutlich mehr Gewebe oder ein deutlich größerer Gewebebereich in den Innenraum eingesaugt werden, bevor die Ansaugwirkung durch Überdeckung des Unterdruck zuführenden Kanals abnimmt.

Bei stark übergewichtigen Patienten kommt es nicht nur zu einer Ansammlung von Fett im Bauchraum sondern auch zu einer Fettablagerung auf dem Perikard. Die durch Drehung der Vorrichtung entstehende Gewebefalte besteht bei diesen Patienten aus Fettgewebe und die vorgeschobene Nadel erreicht nicht mehr den Herzbeutel. Sie dient in diesem Fall ebenfalls zur mechanischen Fixierung des meist schweren Gewebes.

Um dennoch eine zuverlässige Manipulation durchführen zu können sieht die Erfindung vor, dass im Ansaugkopf wenigstens eine weitere Führung für einen weiteren penetrierenden Körper vorgesehen ist. Diese weitere Führung ist bevorzugt relativ zu dem Ansaugkopf schwenkbar ausgebildet.

Mit dieser Anordnung ist ein Zugang zum Herzbeutel selbst dann noch möglich, wenn das Perikard extrem dick ausgebildet ist, beispielsweise durch lokale Fettablagerungen oder durch Vernarbung. Das für den klinischen Einsatz vorgesehene Instrument ist mithin nicht nur bei normalen, sondern auch bei übergewichtigen Patienten einsetzbar.

Der weitere penetrierende Körper kann ein Führungsdraht, eine Nadel, ein Endoskop o.dgl. sein.

Eine noch andere Ausführungsform der Erfindung sieht vor, dass der Ansaugkopf wenigstens eine Ausformung aufweist. Dies kann z.B. eine Stützleiste sein, die im Randbereich des Ansaugkopfes ausgebildet ist.

Der Ansaugkopf kann sich beispielsweise beim Drehen der Vorrichtung auf der Ausformung abstützen, so dass das am Ansaugkopf fixierte Gewebe höher von seinem Untergrund abhebt. Der entstehende Zwischenraum, d.h. der unter dem Gewebe liegende Bereich ist noch besser zugänglich.

Zweckmäßig ist der Ansaugkopf im Bereich der seitlichen Öffnung abgeflacht ausgeführt. Die Vorrichtung kann dadurch flach auf das Gewebe aufgelegt werden.

Am Ansaugkopf der Vorrichtung kann ferner eine Schneidvorrichtung für das Gewebe ausgebildet sein. Dadurch sind weitere Manipulationen möglich.

Ein Verfahren zur Fixierung und/oder Manipulation von Gewebe und/oder darunter liegender Bereiche mit der genannten Vorrichtung, welches nicht Teil der Erfindung ist, weist folgende Schritte auf:
a) Zuführung der erfindungsgemäßen Vorrichtung zu dem zu fixierenden Gewebe;
b) Ansaugen des Gewebes über die seitliche Öffnung der Vorrichtung bis zur Anheftung von Gewebe und mechanische Fixierung des Gewebes;
c) Weitere Anhebung oder Abhebung des Gewebes aus der Lage des Gewebes vor Schritt b);
d) Zuführung des wenigstens einen penetrierenden Körpers zum Gewebe im Abstandsbereich zwischen der Lage des Gewebes vor Schritt b) und dem am weitesten davon beabstandeten Gewebebereich im Innenraum der Vorrichtung.

Damit wird ein zuverlässigeres Verfahren zur Fixierung und nachfolgenden Manipulation erreicht, insbesondere auch für solche Fälle - wie oben beschrieben - in welchen das Gewebe sehr dickwandig ist und eine Manipulation im Innenraum der Vorrichtung keine Eröffnung der darunter liegenden Bereiche ermöglicht.

Eine vorteilhafte erste Ausführungsform des Verfahrens ist durch Variation von Schritt c) gegeben, indem die weitere Anhebung oder Abhebung durch Drehung des länglichen Hohlkörpers um dessen Längsachse und damit auch des mit dem Hohlkörper im Bereich der seitlichen Öffnung verbundenen Gewebes erfolgt. Durch diesen Schritt wird der Manipulationsbereich von den unter dem Gewebe liegenden, empfindlichen Bereichen (z.B. Herzmuskel) weggedreht und damit bei der nachfolgenden Manipulation geschützt.

Eine vorteilhafte zweite Ausführungsform des Verfahrens sieht die Variation von Schritt d) vor, indem die Zuführung von Manipulationselementen zum Gewebe im Abstandsbereich zwischen der Lage des Gewebes vor Schritt b) und dem am weitesten davon beabstandeten Gewebebereich im Innenraum der Vorrichtung außerhalb des vakuumzuführenden Bereiches des Innenraumes des Hohlkörpers vorsieht. Dadurch kann eine Manipulation auch außerhalb des Innenraumes des Hohlkörpers im Bereich der seitlichen Öffnung erfolgen, was insbesondere bei dickwandigen Geweben eine zuverlässigere Eröffnung des Bereiches unterhalb des Gewebes ermöglicht.

Vorteilhaft ist auch die Kombination der ersten Ausführungsform des Verfahrens mit einer weiteren Ausführungsform der Vorrichtung, bei welcher im Bereich der seitlichen Öffnung außen am Führungskörper oder am Hohlkörper der Vorrichtung eine Erhebung in Form einer Kante oder Wandung (im Folgenden auch Bodenlippe genannt) angebracht ist. Dadurch wird zum einen die Reibung zwischen Gewebe und der Vorrichtung erhöht und darüber hinaus dient diese Bodenlippe dazu nach einer entsprechenden Drehung eine Barriere zwischen dem Manipulationsbereich und dem unter dem angehobenen Gewebe gelegenen empfindlichen Bereichen zu bilden.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine Draufsicht auf eine erfindungsgemäße Vorrichtung;
- Fig. 2: einen Schnitt etwa in der Längsebene durch den Ansaugkopf der Vorrichtung;
- Fig. 3: eine Draufsicht auf eine andere Ausführungsform eines Ansaugkopfes der Vorrichtung;
- Fig. 4: einen Schnitt etwa senkrecht zur Längsebene der Vorrichtung durch den Ansaugkopf von Fig. 2;
- Fig. 5: einen etwa senkrecht zur Längsebene gelegenen Schnitt durch ein Handstück zur Steuerung des Ansaugkopfes;
- Fig. 6a: einen Ansaugkopf mit eingesaugtem Gewebe;
- Fig. 6b: den Ansaugkopf nach Rechtsdrehung um ca. 90°;
- Fig. 6c,d: den Ansaugkopf mit eingesaugtem Gewebe mit besonders dicker Wandung;
- Fig. 7a: eine Ausführungsform der Vorrichtung mit einer im Ansaugkopf befindlichen Führung für eine Schneidevorrichtung, wobei Gewebe in den Ansaugkopf eingesaugt wird;
- Fig. 7b: den Ansaugkopf nach Drehung um 90°;
- Fig. 7c: den Ansaugkopf nach dem Abtrennen des eingesaugten Gewebes;
- Fig. 8a: einen Schnitt etwa in der Längsebene des Ansaugkopfes von Fig.7;
- Fig. 8b: eine Draufsicht des Ansaugkopfes von Fig.7;
- Fig. 8c: einen Schnitt etwa senkrecht zur Längsebene des Ansaugkopfes von Fig.7;
- Fig. 9a,b: eine weitere Ausführungsform einer erfindungsgemäßen Vorrichtung;
- Fig. 10: eine flexible Ausführungsform des Hohl- oder Führungskörpers mit unterschiedlichen Ansaugköpfen und Manipulationsvorrichtungen;
- Fig. 11: schematisch das Vorgehen bei minimal-invasiven Manipulationen im oder am Herzbeutel;
- Fig. 12a: einen Querschnitt durch einen Ansaugkopf einer anderen Ausführungsform der erfindungsgemäßen Vorrichtung;
- Fig. 12b: den Ansaugkopf von Fig. 12a im Einsatz;
- Fig. 12c: einen Querschnitt durch einen Ansaugkopf einer noch anderen Ausführungsform der erfindungsgemäßen Vorrichtung;
- Fig. 12d: den Ansaugkopf von Fig. 12c im Einsatz;
- Fig. 12e: einen Querschnitt durch ein Handstück zur Steuerung des Ansaugkopfes der Fig. 12c;
- Fig. 12f: einen Längsschnitt durch den Ansaugkopf von Fig. 12c, und
- Fig. 12g: eine Draufsicht auf den Ansaugkopf von Fig. 12c.

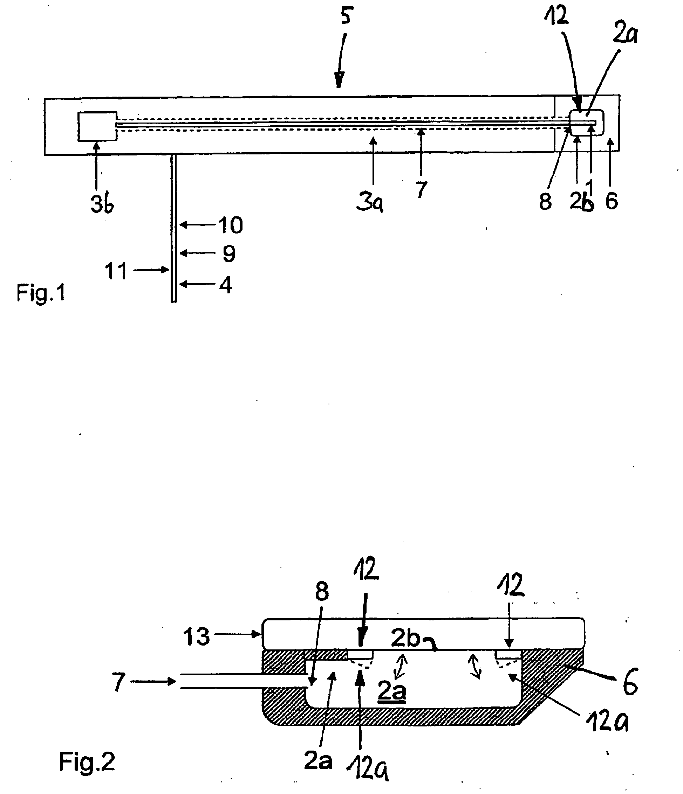

Die in Fig. 1 gezeigte Vorrichtung dient zum Fixieren und Manipulieren von solchen Geweben, welche durch Ansaugen zumindest temporär fixierbar sind. Unter Geweben werden hierbei neben organischen Geweben, z.B. Gewebe von Lebewesen, auch anorganische Gewebe wie Folien oder Textilien verstanden.

Die Vorrichtung dient insbesondere auch zur Fixierung und Manipulation von Geweben, die empfindliche Organe umgeben, wie z.B. das Perikard beim Menschen.

Die Vorrichtung und das mit der Vorrichtung auszuführende Verfahren, welches nicht Teil der Erfindung ist, sind mithin für solche Fallkonstellationen vorteilhaft, wo mehrere Gewebe übereinander liegen oder wo das äußere Gewebe über einem zu manipulierenden Gewebe oder Organ angeordnet ist, das gegenüber mechanischen Manipulationen sehr empfindlich ist.

Ganz besonders vorteilhaft ist die Erfindung für die Erschließung des Perikardinnenraumes, d.h. des Herzbeutels, insbesondere in den Fällen, in denen das Perikard stark verdickt ist oder in denen sich das Perikard während der gewünschten Manipulation bewegt oder in denen ein im Innenraum befindliches Objekt, beispielsweise ein weiteres Gewebe oder Organ, sich ebenfalls bewegt oder sehr empfindlich gegenüber der gewünschten Manipulation ist.

In jedem Fall ermöglicht die erfindungsgemäße Vorrichtung den Zugang in den Herzbeutel nach Fixierung und Anhebung des Perikards durch Anheftung an die Vorrichtung, weshalb die Vorrichtung auch als "Attachlifter" bezeichnet werden kann.

Die erfindungsgemäße Vorrichtung besteht aus einem länglichen Führungskörper, der am unteren Ende einen Anschluss für einen sich von da an erstreckenden Kanal zur Zuführung eines Vakuums in einen Innenraum aufweist. Der Innenraum liegt in einem Ansaugkopf am distalen Ende des Führungskörpers. Der Ansaugkopf hat in einem seitlichen Bereich eine Öffnung, die den Innenraum begrenzt. Die Öffnung im seitlichen Bereich des Führungskörpers ist zur Aufnahme von Gewebe ausgeformt. Sie hat randseitig Mittel zur mechanischen Fixierung des Gewebes, wobei es sich bei den Mitteln um Spann- oder Klemmelemente handelt. Diese bilden in Form von separaten Lippen oder in Form eines umlaufenden Randes einen Öffnungsquerschnitt, der kleiner ist als der Querschnitt des von der Öffnung begrenzten Innenraums. Die Spann- oder Klemmelemente bilden mithin einen Rand, der in die Ebene der Öffnung des Innenraums hineinragt, so dass sich Hinterschneidungen oder Überhänge bilden.

Die Spann- oder Klemmelemente (Überhänge) sind aus elastischem Material gefertigt, so dass sich die von den Klemmlippen begrenzte Öffnung beim Einsaugen des Gewebes zunächst erweitert. Sobald jedoch eine externe Kraft auf das Gewebe einwirkt, indem z.B. an dem Gewebe gezogen wird, verkleinert sich der von den Lippen oder dem elastischen Rand begrenzte Öffnungsquerschnitt. Das Gewebe wird von den Lippen bzw. dem Rand festgeklemmt, selbst wenn die Vakuumerzeugung unterbrochen wird. Die Mittel zur mechanischen Fixierung des Gewebes erzeugen mithin eine Rückhaltekraft, die maßgeblich zur Funktionalität der Vorrichtung beiträgt.

Die Anordnung der seitlichen Öffnung mit den Spann- oder Klemmelemente und des darunter befindlichen Innenraums ist so gewählt, dass sich für das durch die Öffnung in den Innenraum eingesaugte Gewebe so genannte unter sich gehende Stellen (Hinterschneidungen) ergeben, welche das einmal eingesaugte Gewebe zusätzlich zur Ansaugkraft des Vakuums fixieren. Der Vorteil besteht in einem mechanischen Festhalten des eingesaugten Gewebes, wenn ein Zug nach außen ausgeübt wird. Vorteilhaft sind hierbei die Klemmlippen aus flexiblem Material, wodurch das ein eingesaugte Gewebe selbst mit unregelmäßiger Oberfläche in der Ansaugöffnung abgedichtet wird.

Beim Einsaugen des Gewebes wird die von den Spann- oder Klemmelementen begrenzte Öffnung vergrößert. Tritt hingegen eine Zugkraft nach außen auf, wird die Öffnung verkleinert, weil das Gewebe versucht, die elastischen Elemente nach außen zu ziehen. Das Gewebe wird zwischen den Spann- oder Klemmelementen festgehalten und zudem abgedichtet, was insbesondere dann von Vorteil ist, wenn man die Vorrichtung zum Abheben des Gewebes von dem darunter liegenden Organ dreht.

Vorteilhaft ist auch, dass die Öffnung, die von den flexiblen Spann- oder Klemmelementen begrenzt wird, variabel ist. Ein solcher Ansaugkopf kann daher bei Krankheitsbildern mit unterschiedlicher Gewebedicke eingesetzt werden.

Überraschenderweise wurde gefunden, dass sich mit der erfindungsgemäßen Vorrichtung eine deutlich höhere Anheftung bzw. Fixierung erzielen lässt, welche auch die Fixierung von sehr dickwandigem und damit starrem Gewebe und darüber hinaus eine stets zuverlässige Anhebung des Gewebes (auch im Sinne einer Loslösung von darunter liegenden Bereichen) ermöglicht.

Eine weitere Erhöhung der Fixierwirkung ist erzielbar, wenn die Klemmlippen, nachdem diese durch eingesaugtes Gewebe nach innen geneigt wurden, in dieser Stellung arretiert werden (nicht dargestellt).

Die Spann- oder Klemmelemente können auch aktiv beweglich sein. Sie können z.B. im Volumen oder in ihrer Ausdehnung in einer oder mehr Raumrichtungen veränderliche Körper bilden. Diese können etwa durch Seilzüge, elektrische oder fluid- oder gas-hydraulische Aktoren oder anderweitige aktivierbare Elemente gesteuert werden oder als elektrische oder magnetische oder fluid- oder gas-hydraulische Elemente ausgeführt sein.

In einer weiteren (ebenfalls nicht dargestellten) Ausführungsform weist die erfindungsgemäße Vorrichtung eine Regeleinrichtung auf, welche durch Kraft- oder Neigungserfassungselemente an den beweglichen oder aktiv beweglichen Spann- oder Klemmelementen und entsprechende Regelalgorithmen so ausgelegt ist, dass das angelegte Vakuum in Abhängigkeit von der Kraft des angesaugten Gewebes auf die Klemmlippen oder in Abhängigkeit von der Neigung der Klemmlippen das Vakuum variiert. Oder man variiert das Vakuum und die Neigung der Klemmlippen derart, dass das Gewebe kontinuierlich oder in Schritten tief in die seitliche Öffnung eingesogen wird. Dabei kann auch eine Neigungsverstellung der Klemmlippen um bis zu 360° vorgesehen sein.

Die Vorrichtung ist mit seiner seitlichen Öffnung an das Gewebe heranführbar. Nach erfolgreicher Anheftung, welche z.B. durch die in DE-A1-103 37 813 offenbarte Vorrichtung zur Unterdrucküberwachung und -anzeige zuverlässig erkannt werden kann, kann ein in dem Führungskörper geführtes Instrument zur Manipulation oder Punktion des am Ansaugkopf fixierten Gewebes aktiviert werden. Im Falle einer Punktion des Gewebes ist der Bereich unterhalb des Gewebes, z.B. der Perikardinnenraum für weitere Manipulationen, wie dem Zu- oder Abführen von Flüssigkeiten oder Materialien (z.B. Elektroden oder pharmazeutische Wirkstoffe) eröffnet.

In einer vorteilhaften Ausführungsform, weist die Vorrichtung außerhalb des Führungsköpers oder des Inneren des Hohlkörpers weitere Mittel zur Zuführung von Manipulationselementen oder optischen Elementen oder weiteren in der Medizintechnik oder Technik bekannten Elementen in den Bereich der Anheftung des Gewebes an die erfindungsgemäße Vorrichtung auf. Durch diese räumliche Trennung wird erreicht, dass die Vorrichtung keine Vakuumdichtungen zur Führung von Elementen im Inneren des Führungskörpers benötigt. Darüber hinaus ergibt sich der Vorteil, dass der Bereich der Anheftung vom Bereich der Manipulation oder optischen Erfassung räumlich getrennt wird. Dies ist insbesondere für sehr dickwandige Gewebe vorteilhaft, da diese den Innenraum des Hohlkörpers häufig komplett mit der Wandung des Gewebes ausfüllen und somit eine Punktion des Gewebes im Innenraum nicht zu einer Eröffnung der unter der Wandung liegenden Bereiche führt.

Dieser Umstand ist insbesondere in der medizinischen Anwendung der Vorrichtung anzutreffen, wo etwa im Bereich der minimal-invasiven Chirurgie, der Innenraum nicht beliebig voluminös ausgeführt sein kann.

Fig.1 stellt die wesentlichen Bestandteile der erfindungsgemäßen Vorrichtung dar. Sie hat einen Führungskörper 5 mit einem Ansaugkopf 6. Dieser weist einen Innenraum 2a auf, der von einer seitlichen Öffnung 2b begrenzt wird. In dem Führungskörper ist ein Kanal 7 ausgebildet, die mit einer Öffnung 8 im Innenraum 2a endet. Der Kanal 7 dient als Mittel zum Einbringen eines Vakuums in den Innenraum 2a. Dazu ist ferner eine Unterdruckquelle 4 an den Kanal 7 angeschlossen.

Seitlich und parallel neben dem Kanal 7 ist ein Führungskanal 3a für einen penetrierenden Körper 1 ausgebildet. Dieser kann eine Nadel, eine Schere, ein Messer, eine Zange, ein Draht oder eine Elektrode sein. Er wird von einer optionalen Steuervorrichtung 3b betätigt und/oder gesteuert.

Optional weist die Vorrichtung eine Anheftungserfassungsvorrichtung 9, wie Sie in DE-A1-103 37 813 beschrieben ist, auf. Diese kann einen Drucksensor umfassen sowie eine Anzeigevorrichtung 11, um anzuzeigen, ob der erforderliche Ansaugdruck im Innenraum 2a erreicht ist. Auf dieses Weise kann der Bediener der Vorrichtung erkennen, ob eine ausreichende Fixierung durch das Vakuum erreicht ist. Der Drucksensor 9 kann auch innerhalb des Ansaugkopfes 6 oder im Bereich darunter in der Vorrichtung angeordnet sein. Um eine Punktion oder anderweitige Manipulation, z.B. eine Bestrahlung zu ermöglichen, wird der penetrierende Körper 1 mittels der Steuervorrichtung 3b, in Richtung seiner Längsachse innerhalb der Führung 3a vorgeschoben, rückgeschoben und/oder gedreht. Wichtig dabei ist, dass der penetrierende Körper 1 nicht innerhalb des Vakuumkanals 7 liegen, sondern in einem separaten Kanal 3a angeordnet ist, so dass zwischen dem penetrierendem Körper 1 und dem Innenraum 2a des Ansaugkopfes 6 ein räumlicher Abstand entsteht. Überdies muss die Führung 3a des penetrierenden Körpers 1 nicht mit Vakuumdichtungen versehen werden, was sich günstig auf die Herstellkosten und auf die Zuverlässigkeit der Vorrichtung auswirkt.

Der penetrierende Körper 1 wird vom proximalen Ende in die Vorrichtung eingeführt. Es ist eine Arretierung vorgesehen, welche den penetrierenden Körper in einer Position "vor dem Durchstechen" eines Gewebes oder Materials hält. Die Arretierung kann in Form einer einfachen mechanischen Vorrichtung, z.B. in Form einer Klemmung, ausgeführt sein. Nach der zuverlässigen Erkennung der Anheftung des Gewebes oder des Organs kann der penetrierende Körper 1 in dieses eingeführt werden. Das Handstück zur Steuerung des Hohl- oder Führungskörpers oder nur des Ansaugkopfes ist nicht dargestellt.

Als Unterdruckquelle 4 wird vorzugsweise eine kontinuierlich saugende Pumpe (ohne Nachregelung des Druckes bei Druckveränderung) oder eine kontinuierlich mit konstanter Leistung arbeitende Unterdruckquelle eingesetzt, um eventuelle Undichtigkeiten bei der Anheftung erfassen zu können. Die Erkennung der Anheftung wird durch vorherige Eichung oder Kalibrierung mittels Druckmessung bei verschlossenem Ansaugkopf erreicht.

Fig.2 zeigt einen Schnitt der erfindungsgemäßen Ausführung, etwa in der Längsebene des Führungs- oder Hohlkörpers 5 der Vorrichtung, im Bereich des Ansaugkopfes 6. Deutlich zu erkennen ist der unter dem Bereich der Öffnung 2b liegende Innenbereich 2a des Ansaugkopfes 6 mit den überhängenden Spann- oder Klemmelementen 12, die gegenüber dem Rand der Öffnung 2b Hinterschneidungen bilden. Die Spann- oder Klemmelemente 12 sind als bewegliche Klemmlippen 12a ausgebildet, die zu einem größeren oder kleineren Öffnungsquerschnitt 12c führen können. Zu erkennen ist auch, dass sich die Öffnung 8 zum Vakuum zuführenden Kanal 7 in einem Abstand a zum linken Rand der oberen Öffnung 2b befindet. Diese Ausführung sorgt dafür, dass auch ein relativ großer Gewebebereich eingesaugt werden kann, bevor die Ansaugwirkung durch Überdeckung der Vakuumöffnung 8 abnimmt.

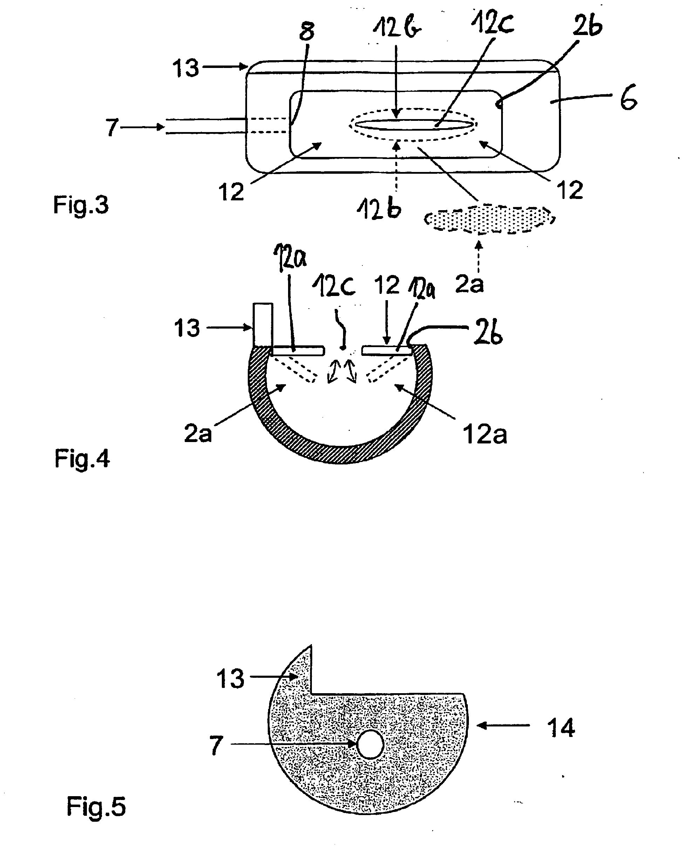

Fig.3 zeigt einen Ansaugkopf in einer Draufsicht, bei dem die Spann- oder Klemmelemente 12 einen umlaufenden Rand 12b bilden. Auch hierbei entstehend Überhänge mit Hinterschneidungen, die das Gewebe 15 im Ansaugkopf 6 fixieren.

Fig.4 zeigt den Schnitt etwa senkrecht zur Längsebene des Ansaugkopfes aus den Fig.2,3. Dieser Ansaugkopf weist im Bereich der Öffnung 2a, 2b eine Ausformung 13, hier in Form einer Wandung auf. Durch diese Ausformung wird u.a. die Reibung des Gewebes an der Vorrichtung erhöht und damit die fixierende Wirkung, z.B. bei einer Drehung, verstärkt. Vor allem wird eine Barriere zwischen Manipulationsvorrichtung und Gewebe geschaffen daher auch Schutzlippe genannt.

Fig.5 zeigt den Schnitt etwa senkrecht zur Längsebene eines Handstücks 14 zur Steuerung des Hohl- oder Führungskörpers 5 oder bei mehrteiliger Form der Vorrichtung auch des Ansaugkopfes 6 alleine und zwar mit einer Ausformung 13, wie diese auch am Ansaugkopf vorgesehen ist, so dass der Bediener/Operateur immer eine direkte Rückmeldung zur Lage des Ansaugkopfes erhält.

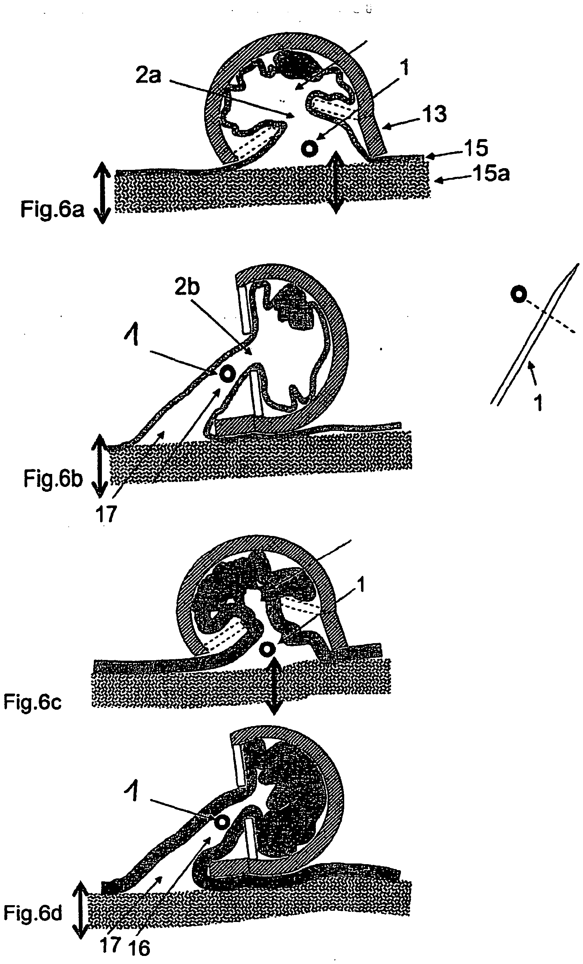

Fig.6 a, b zeigen einen Ansaugkopf nach Fig.4 in Aktion und zwar in 6a nach Ansaugung von Gewebe 15, das sich von dem darunter liegenden verschieblichen Gewebe/Organ 15a abheben lässt. Fig. 6a zeigt den Schritt b) des Verfahrens und Fig.6b den Zustand nach der Drehung um ca. 90° nach rechts (nach Schritt c2) des Verfahrens). Hierbei entsteht ein Manipulationsbereich 16 im Abhebungsbereich 17. Eine weitere vorteilhafte Wirkung wird sichtbar, welche darin besteht, dass die Ausformung 13 eine Barriere zwischen dem Manipulationsbereich 16 und dem Gewebe/Organ 15a schafft. Außerdem wird in Fig.6c,d gezeigt, dass auch bei dickwandigem Gewebe, das den Ansaugkopf vollständig ausfüllen kann, ein Abhebungsbereich 17 für eine Manipulation im Manipulationsbereich 16 geschaffen wird.

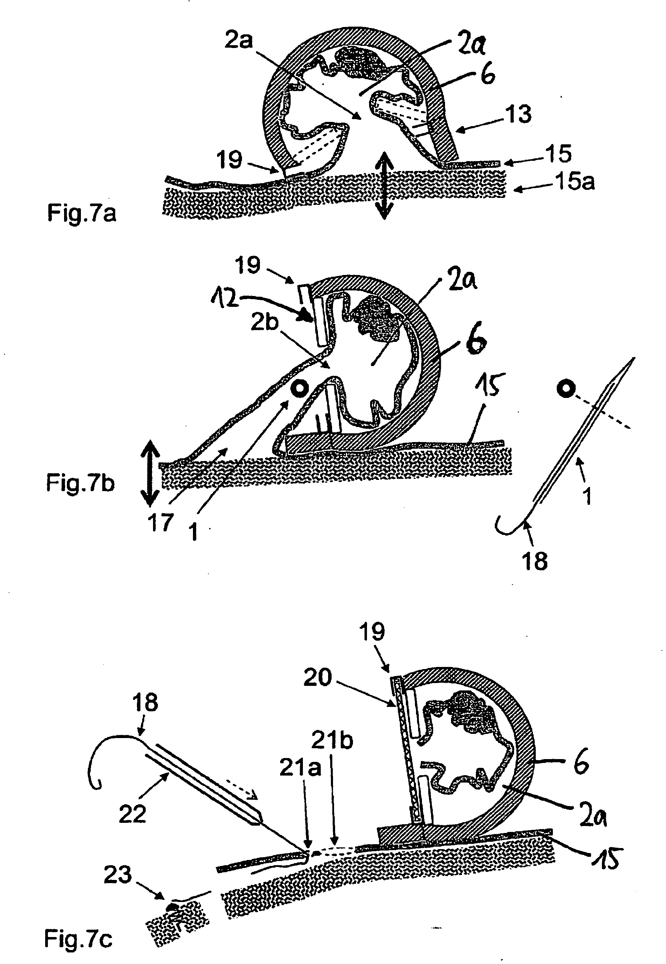

Fig.7 a, b, c zeigen eine weitere vorteilhafte Ausführungsform der Vorrichtung mit einer am Ansaugkopf befindlichen Schneidevorrichtung, Schlinge oder anderen Manipulationsvorrichtungen. In a) wird das Gewebe in den Ansaugkopf eingesaugt, in b) erfolgt nach einer Rechtsdrehung eine Gewebemanipulation im Manipulationsbereich 16, z.B. Einstechen einer Nadel 1 mit in der Nadel liegendem Führungsdraht 18. Diese vorteilhafte Ausführungsform des penetrierenden Körpers ist im Querschnitt als Punkt im Manipulationsbereich dargestellt - zur Verdeutlichung ist die Nadel auch im Längsschnitt dargestellt. In c) wird das im Ansaugkopf befindliche Gewebe durch eine in einer Führung 19 laufenden Schneidevorrichtung z.B. Messer 20, Schlinge abgetrennt. In c) ist schematisch auch das weitere Vorgehen beim Zugang durch das Perikardgewebe in den darunter liegenden Herzbeutel dargestellt. Nachdem die Nadel das Perikard durchstochen hat, wird der Führungsdraht in den Herzbeutel vorgeschoben. Auf dem Führungsdraht wird ein Dilatator 22 in Richtung der durch die Nadel geschaffenen Öffnung 21a geschoben. Durch Aufweiten der Öffnung reißt die schmale Verbindung/Steg zu der durch die Schneidevorrichtung entstandenen Öffnung 21 b auf. Mit diesem Verfahren wird eine für darauf folgende Manipulationen erforderliche große Öffnung im Perikard mit in der Öffnung liegendem und in den Herzbeutel vorgeschobenem Führungsdraht 18 geschaffen. Ein vorteilhaftes sich daraus ergebendes Verfahren ist die epikardiale Implantation einer Schraubelektrode 23 z.B. für Herzschrittmacher (schematisch eingezeichnet).

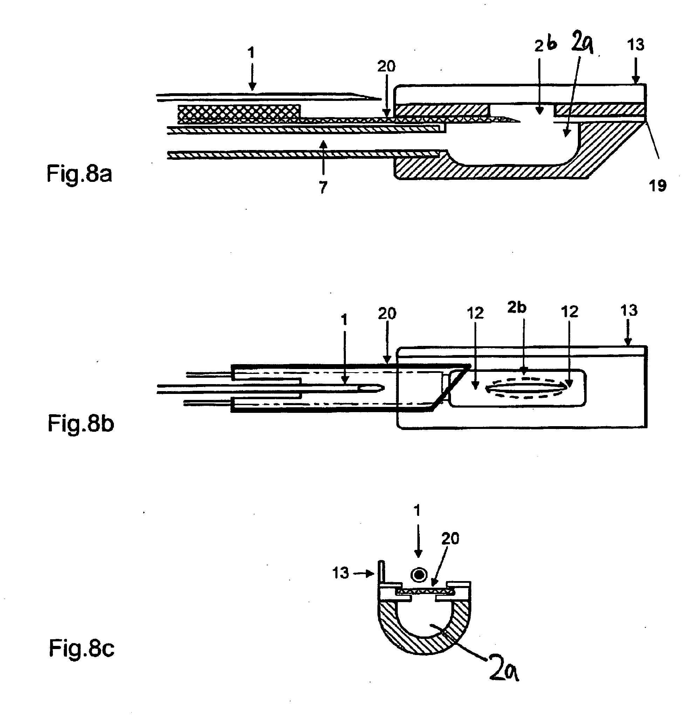

Fig.8 a, b, c zeigt einen Schnitt in der Längsebene, eine Draufsicht und einen Schnitt etwa senkrecht zur Längsebene einer möglichen Ausführungsform wie in Fig.7 beschrieben.

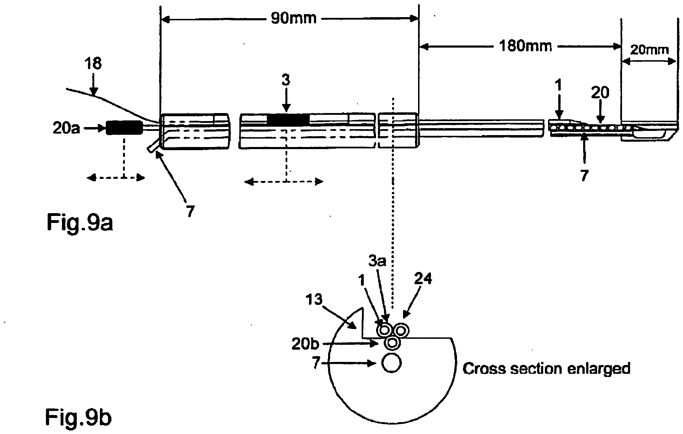

Fig.9 zeigt ein vorteilhaftes Ausführungsbeispiel mit einer möglichen Bemaßung in mm für den Zugang in den Herzbeutel, mit einer Nadel als penetrierenden Körper 1 und zugehöriger Steuervorrichtung 3 und optional einer Schneidevorrichtung in Form eines verschiebbaren Messers 20 mit zugehöriger Steuervorrichtung 20a und einer Führung für ein Endoskop 24. a) stellt einen Schnitt etwa in der Längsebene der Vorrichtung und b) einen Schnitt etwa senkrecht zur Längsebene in Höhe des Handstückes außerhalb der Steuervorrichtung 3 dar die Messerführung ist mit 20b bezeichnet. In a) sind die Führungen für Nadel und Messer nicht eingezeichnet, die eine Verletzung des Gewebes vor der eigentlichen Gewebemanipulation verhindern.

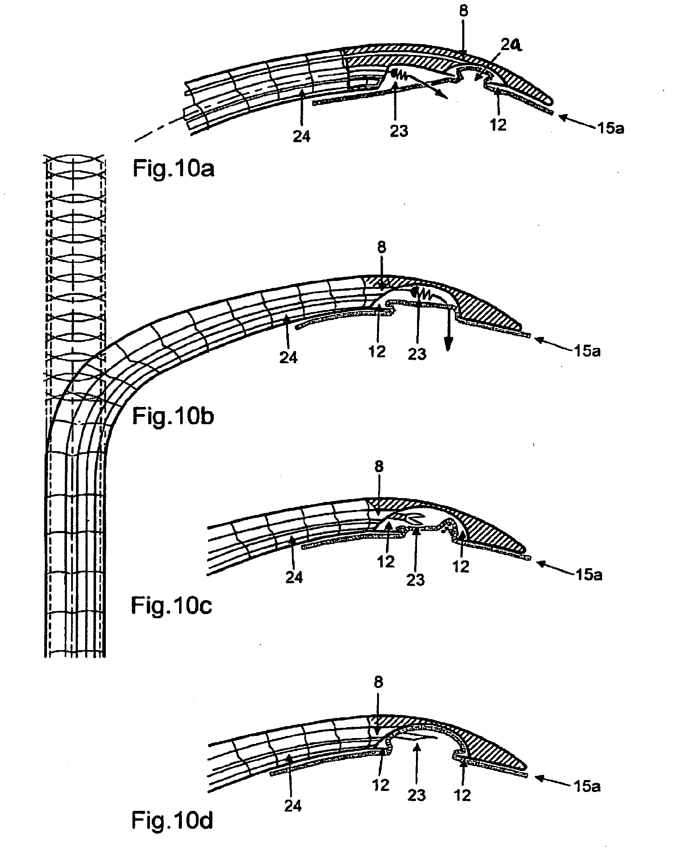

Fig.10 zeigt eine flexible Ausführungsform des Hohl- oder Führungskörpers mit innen liegenden, aber getrennten und damit beabstandeten Kanälen für Vakuum und Führung der möglichen manipulierenden oder penetrierenden Körper. Vorteilhafte Manipulationsvorrichtungen sind in: a) und b) Elektroden die Gewebefixierung durch Ansaugung erleichtert das Eindrehen einer Schraubelektrode in das Gewebe/Organ; c) Schneidevorrichtung und d) penetrierender Körper in Form einer Nadel. Vorteilhafte Manipulationsorte sind in: a) außerhalb des Ansaugkopfes und b) - d) innerhalb des Ansaugkopfes. In b)-d) verlaufen die Manipulationsvorrichtungen im Vakuumkanal. In diesen Ausführungsbeispielen ist keine weitere Anhebung oder Abhebung des Gewebes z.B.

Drehung aus Fig. 6) im Sinne des Verfahrens erforderlich. Die Ansaugköpfe weisen Überhänge Klemmlippen 12 mit unter sich gehenden Bereichen 12a auf. In b) wird die Einschraubelektrode durch den Ansaugkopf geführt und es gibt daher in diesem Bereich keinen Überhang. Vorteilhaft ist ein Lichtleiter Endoskop 24 zur Beobachtung der gewebemanipulierenden Körper 23. In Fig. 10 ist ein Gewebe/Organ mit 15a und ein abhebbares/verschiebliches Gewebe mit 15 bezeichnet.

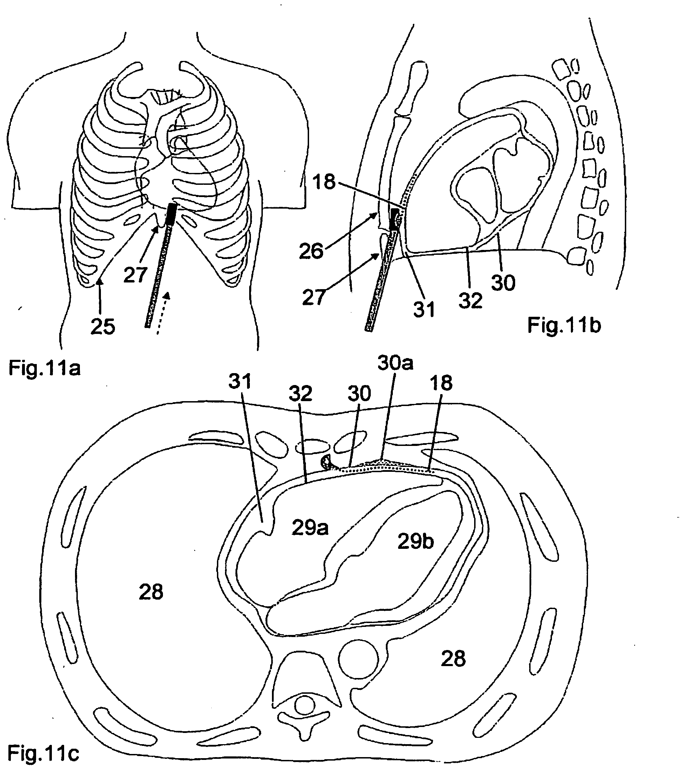

Fig.11 a, b, c zeigt schematisch ein Verfahren unter Einsatz der erfindungsgemäßen Vorrichtung, hier mit einem flexiblen Führungs- oder Hohlkörper, bei minimat-invasiven Manipulationen im Herzbeutel. Darin sind a) Draufsicht, b) Längsschnitt, c) Querschnitt. Das Verfahren ist gekennzeichnet durch die Schritte: Der Ansaugkopf wird nach Zugang in den Brustraum unter dem Processus Xiphoideus 27 auf das Perikard 30 gelegt, das Perikard punktiert und ein Führungsdraht in den darunter liegenden Herzbeutel 31 vorgeschoben. Anschließend können weitere Manipulationen ausgeführt werden. Eine vorteilhafte Manipulation ist die Implantation einer Schraubelektrode 23 (z.B. für Herzschrittmacher schematisch an der Spitze der linken Herzkammer eingezeichnet). Im Falle einer Implantation von Elektroden auf der Herzoberfläche Epikard 32 kommt es zu keiner Einschränkung der Lungenfunktion. Im vorteilhaften Ausführungsbeispiel des Herzens entspricht das abzuhebende verschiebliche Gewebe 15 aus Fig.6 und 7 dem Perikard 30. Das darunter liegende Gewebe/Organ 15a entspricht dem Epikard 32 und Herzmuskel.

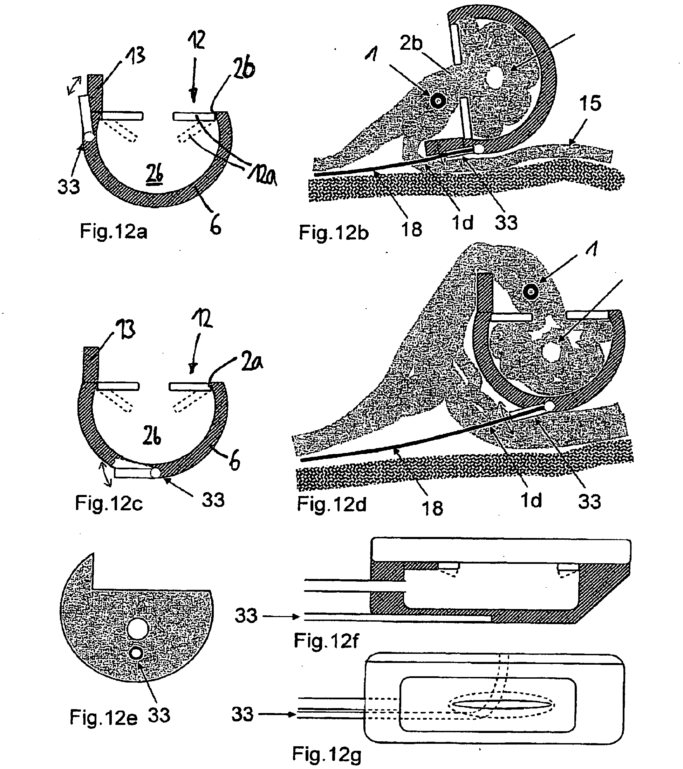

Im Ausführungsbeispiel der Fig. 12 erfolgt eine Ansaugung wie bereits beschrieben mit anschließender Drehung der Vorrichtung um ca. 90° gefolgt vom Vorschieben der Nadel. Da es sich in diesem Fall um ein stark verfettetes Perikard handelt, besteht die Gewebefalte überwiegend aus Fettgewebe und die Nadel erreicht nicht den Herzbeutel. Der Ansaugkopf mit dem durch die Nadel fixierten Gewebe wird daher um ca. 90° Grad weiter gedreht. Hierbei wird das Gewebe am Ansaugkopf durch die in das Gewebe eingestochene Nadel und den Zug des an mehreren Stellen an Organen festgewachsenen Perikards "festgektemmt". Diese Klemmung erfordert vorzugsweise das Einstechen einer Nadel oder allgemein einer anderen Fixiervorrichtung. Die Unterdruck-vermittelte Ansaugung ist hierbei nicht ausreichend und das Gewebe würde sich bei einer Drehung um ca. 180° vom Ansaugkopf wieder ablösen. Anschließend wird senkrecht zum festgeklemmten Gewebe vorzugsweise ein Draht, Dilatator, Nadel etc in das festgeklemmte Gewebe vorgeschoben. Wird der Draht hierbei nicht parallel zur Perikardoberfläche, sondern schräg zur Herzoberfläche geneigt, vorgeschoben, durchdringt er das Perikard bei jeder Dicke des Gewebes. Mit dieser Anordnung gelingt es daher ein Perikard mit unterschiedlicher Dicke zu perforieren. Erreicht der Dilatator die andere Seite des Perikards, kommt es nicht zu einer Verletzung des häufig dicht beabstandeten Epikards, da der vorzugsweise verwendete flexible Draht (vorzugsweise flexibler Führungsdraht mit spiraligem Aufbau) im Herzbeutel keine mechanische Führung mehr hat, die eine gerichtete Bewegung vorgibt. Der flexible Draht daher nicht in das Epikard einstechen sondern sich im Herzbeutel fortbewegen. Auch erfolgt die Vorwärtsbewegung des flexiblen Drahtes in sehr kleinen Schritten und der Operateur spürt das Überwinden des Widerstandes beim Durchstechen der äußeren bindegewebigen Seite gefolgt vom Durchstechen der inneren Seite bindegewebigen Seite des Perikard. Sollte sich der Draht im Fettgewebe weiterbewegen, so wird dies erkannt, der Draht wird zurückgezogen und nach einer Drehung wieder vorgeschoben. Während dieses Vorgehens bleibt das Perikard fest an die Vorrichtung angeklemmt. Dieses Ausführungsbeispiel 1 erfordert, dass das Perikard straff gespannt ist und daher an die Vorrichtung nach Drehung um 180° angeklemmt wird.

Im Falle eines weniger straff gespannten Gewebes gedehnt (z.B. als Folge eines vorausgegangen Perikardergusses) wird der Ansaugkopf mit dem durch die Nadel fixierten Gewebe nicht nur um 180° sondern vorzugsweise um ca. 360° Grad gedreht. Hierbei wird das Gewebe am Ansaugkopf durch das Aufwickeln stark festgeklemmt. Das Verfahrens erfordert, dass genügend Gewebe (2* 2*π*Radius des Ansaugkopfes) für das Aufwickeln zur Verfügung steht.

Ein weiteres (nicht dargestelltes) Ausführungsbeispiel kommt bei einem nicht straff gespannten Gewebe zum Einsatz, wobei hier eine Drehung nur um 180° erfolgt. Um eine ausreichende Klemmung zu erreichen, wird ein Kopfteil mit 2 um 180° versetzten Ausnehmungen oder Ansaugköpfen verwendet. Es wird zunächst der 1. Ansaugkopf mit angesaugtem Gewebe um ca. 90° Grad nach links gedreht und die Nadel in das verdickte Perikard eingestochen. Der Kopf wird anschließend um 90° weiter gedreht und der nun auf dem Perikard liegende 2. Kopf angesaugt, um ca. 90° nach rechts gedreht und die 2. Nadel in das verdickte Gewebe eingestochen. Der Kopf wird dann um ca. 90° nach links gedreht insgesamt 180°). Es ist nun das Gewebe an beiden Seiten des Ansaugkopfes an den Ansaugkopf "angeklemmt" und kann bei der nun folgenden Manipulation, die senkrecht zur Klemmung erfolgt, nicht ausweichen.

Fig. 12 a und b zeigten einen Ansaugkopf, der eine weitere Führung 33 für einen weiteren penetrierenden Körper 18 aufweist. Diese kann gegenüber dem Ansaugkopf 6 verschwenkt werden, so dass ein darin geführter penetrierender Körper 18, z.B. ein Draht senkrecht zum Ansaugkopf 6 in das Gewebe 15 eingeführt werden kann.

In Fig. 12a ist die Führung 33 unterhalb der Ausformung 13 seitlich am Ansaugkopf 6 ausgebildet. In Fig. 12 c ist die Führung 33 gegenüberliegend zur Öffnung 2b ausgebildet.

Die Erfindung ist nicht auf eine der vorbeschriebenen Ausführungsformen beschränkt, sondern in vielfältiger Weise abwandelbar. Man erkennt jedoch, dass eine Vorrichtung zum Punktieren oder/und anderen Manipulationen von organischem oder anorganischem Gewebe oder darunter liegender Bereiche, mindestens einen länglichen Hohl- oder Führungskörper 5) umfasst, z.B. in Form eines starren oder flexiblen Rohres oder Stabes. Die Vorrichtung hat ferner Mittel zur Führung mindestens eines penetrierenden Körpers 1) sowie Mittel zur Zuführung, z.B. in Form eines Kanals 7), von Vakuum in einen Innenraum des Körpers 5), welcher mindestens eine seitliche Öffnung 2) aufweist. Um die Fixierung des Gewebes zu verbessern, ist die Öffnung im seitlichen Bereich des Hohlkörpers zur Ausbildung eines Ansaugkopfes 6) so ausgeformt oder ausformbar ausgeführt, dass der Querschnitt der Öffnung, etwa durch einen umlaufenden Rand oder einzelne in die Ebene der Öffnung hineinragende Abschnitte oder in anderer Form, kleiner ausgeführt ist, als der darunter, parallel zur Ebene der Öffnung liegende Querschnitt des Innenraumes der Vorrichtung

### Bezugszeichenliste

- 1: penetrierender Körper
- 2a: Innenraum
- 2b: seitliche Öffnung
- 3a: Führung
- 3b: Steuervorrichtung
- 4: Unterdruckquelle
- 5: Führungskörper
- 6: Ansaugkopf
- 7: Vakuumkanal
- 8: Vakuumöffnung
- 9: Anheftungserfassungsvorrichtung
- 10: Drucksensor
- 11: Anzeigevorrichtung
- 12: mechanisches Fixiermittel
- 12a: elastische Lippe
- 12b: elastischer Rand
- 12c: Öffnungsquerschnitt
- 13: Ausformung, Boden-/Schutzlippe
- 14: Handstück
- 15: Gewebe
- 16: Manipulationsbereich
- 17: Abhebungsbereich
- 18: Führungsdraht
- 19: Führung für Schneidevorrichtung
- 20: Messer oder Schlinge
- 21: Gewebeöffnung
- 22: Dilatator
- 23: Gewebemanipulierender Körper
- 24: Endoskop
- 25: Rippen
- 26: Stemum
- 27: Processus Xiphoideus
- 28: Lunge
- 29: Herzkammem (a, rechts; b, links)
- 30: Perikard
- 31: Perikardhöhle ("Herzbeutet")
- 32: Epikard des Herzmuskels

## Patentansprüche

1. Vorrichtung zur Fixierung und/oder Manipulation von Gewebe (15) und/oder darunter liegender Bereiche (16), mit wenigstens einem das Gewebe (15) und/oder die darunter liegenden Bereiche (16) penetrierenden Körper (1) und mit einem Führungskörper (5) für den penetrierenden Körper (1), wobei der Führungskörper (5)
■ Mittel (3a) zur Führung des penetrierenden Körpers (1) aufweist,
■ endseitig einen Ansaugkopf (6) für das Gewebe (15) hat oder bildet,
■ innerhalb des Ansaugkopfes (6) einen Innenraum (2a) aufweist, mit wenigstens einer im seitlichen Bereich des Führungskörpers (5) ausgebildeten Öffnung (2b) zur Aufnahme des zu punktierenden und/oder zu manipulierenden Gewebes (15), und
■ Mittel (7) aufweist, zum Einbringen eines Vakuums in den Innenraum (2a),
wobei im Bereich der Öffnung (2b) des Ansaugkopfes (6) wenigstens ein Mittel (12) zur
mechanischen Fixierung und/oder Festlegung des Gewebes (15) am Ansaugkopf (6) vorgesehen ist, **dadurch gekennzeichnet, dass** das wenigstens eine Mittel (12) zur mechanischen Fixierung und/oder Festlegung des Gewebes (15) am Ansaugkopf (6) ein elastisches Spann-oder Klemmelement ist.

2. Vorrichtung nach Anspruch 1, **dadurch gekennzeichnet, dass** jedes elastische Spann- oder Klemmelement (12) eine elastische Lippe (12a), Feder oder Zunge bildet, die zumindest teilweise in die Öffnung (2b) hineinragt.

3. Vorrichtung nach Anspruch 1 oder 2, **dadurch gekennzeichnet, dass** das elastische Spann- oder Klemmelement (12) einen umlaufenden elastischen Rand (12b) für die Öffnung (2b) bildet.

4. Vorrichtung einem der Ansprüche 1 bis 3, **dadurch gekennzeichnet, dass** der von den elastischen Spann- oder Klemmelementen (12) begrenzte Öffnungsquerschnitt (12c) kleiner ist als der Querschnitt des von der Öffnung (2b) begrenzten Innenraums (2a).

5. Vorrichtung nach einem der Ansprüche 1 bis 4, **dadurch gekennzeichnet, dass** die elastischen Spann- oder Klemmelemente (12) aktiv beweglich oder veränderbar sind.

6. Vorrichtung nach einem der Ansprüche 1 bis 5, **dadurch gekennzeichnet, dass** die elastischen Spann- oder Klemmelemente (12) mechanisch, elektrisch, magnetisch, hydraulisch oder pneumatisch bewegbar sind.

7. Vorrichtung nach einem der Ansprüche 1 bis 6, **dadurch gekennzeichnet, dass** der penetrierende Körper (1) außerhalb des Ansaugkopfes (6) gleitgeführt ist.

8. Vorrichtung nach einem der Ansprüche 1 bis 7, **dadurch gekennzeichnet, dass** die Mittel (3a) zur Führung des penetrierenden Körpers (1) getrennt von den Mitteln (7) zum Einbringen eines Vakuums in den Innenraum (2a) ausgebildet sind.

9. Vorrichtung nach einem der Ansprüche 1 bis 8, **dadurch gekennzeichnet, dass** im Ansaugkopf (6) wenigstens eine weitere Führung (33) für einen weiteren penetrierenden Körper (18) vorgesehen ist.

10. Vorrichtung nach Anspruch 9, **dadurch gekennzeichnet, dass** die weitere Führung (33) relativ zu dem Ansaugkopf (6) schwenkbar ausgebildet ist.

11. Vorrichtung nach einem der Ansprüche 1 bis 10, **dadurch gekennzeichnet, dass** der Ansaugkopf (6) wenigstens eine Ausformung (13) aufweist, wobei die Ausformung (13) im Randbereich des Ansaugkopfes (6) ausgebildet ist.

12. Vorrichtung nach einem der Ansprüche 1 bis 11, **dadurch gekennzeichnet, dass** das am Ansaugkopf (6) eine Schneidvorrichtung (20) für das Gewebe (15) ausgebildet ist.

## Claims

1. Device for fixation and/or manipulation of tissue (15) and/or regions (16) lying beneath said tissue, comprising at least one body (1) that penetrates the tissue (15) and/or the regions (16) lying beneath said tissue, and comprising a guiding body (5) for the penetrating body (1), the guiding body (5)
• having means (3a) for guiding the penetrating body (1),
• having or forming a suction head (6) for the tissue (15) at the end of said guiding body,
• having an interior (2a) within the suction head (6), with at least one opening (2b) formed in the lateral region of the guiding body (5), for receiving the tissue (15) to be punctured and/or manipulated, and
• having means (7) for creating a vacuum within the interior (2a),
at least one means (12) is provided in the region of the opening (2b) of the suction head (6) for the mechanical fixation and/or immobilization of the tissue (15) at the suction head (6), **characterized in that** the at least one means (12) for the mechanical fixation and/or immobilization of the tissue (15) at the suction head (6) is a flexible tensioning or clamping element.

2. Device according to claim 1, **characterized in that** each flexible tensioning or clamping element (12) forms a flexible lip (12a), spring or tongue, which projects at least partially into the opening (2b).

3. Device according to claim 1 or 2, **characterized in that** the flexible tensioning or clamping element (12) forms a continuous flexible edge (12b) for the opening (2b).

4. Device according to any one of claims 1 to 3, **characterized in that** the opening cross-section (12c) that is bounded by the flexible tensioning or clamping elements (12) is smaller than the cross-section of the interior (2a) that is bounded by the opening (2b).

5. Device according to any one of claims 1 to 4, **characterized in that** the flexible tensioning or clamping elements (12) are actively movable or alterable.

6. Device according to any one of claims 1 to 5, **characterized in that** the flexible tensioning or clamping elements (12) can be moved mechanically, electrically, magnetically, hydraulically or pneumatically.

7. Device according to any one of claims 1 to 6, **characterized in that** the penetrating body (1) is guided by sliding outside of the suction head (6).

8. Device according to any one of claims 1 to 7, **characterized in that** the means (3a) for guiding the penetrating body (1) and the means (7) for creating a vacuum within the interior (2a) are formed separately.

9. Device according to any one of claims 1 to 8, **characterized in that** at least one additional guide (33) for an additional penetrating body (18) is provided in the suction head (6).

10. Device according to claim 9, **characterized in that** the additional guide (33) is slewable relative to the suction head (6).

11. Device according to any one of claims 1 to 10, **characterized in that** the suction head (6) has at least one projection (13), the projection (13) is formed in the edge region of the suction head (6).

12. Device according to any one of claims 1 to 11, **characterized in that** a cutting device (20) for the tissue (15) is formed at the suction head (6).

## Revendications

1. Dispositif pour la fixation et/ou la manipulation de tissus (15) et/ou des régions sous-jacentes (16), avec au moins un corps (1) pénétrant dans le tissu (15) et/ou dans les zones sous-jacentes (16), et avec un corps de guidage (5) pour le corps pénétrant (1), dans lequel le corps de guidage (5)
- comporte des moyens (3a) pour le guidage du corps pénétrant (1),
- possède ou forme une tête d'aspiration (6) côté extrémité, pour le tissu (15),
- comporte un espace intérieur (2a) dans la tête d'aspiration (6), avec au moins une ouverture (2b) formée dans la région latérale de la tête de guidage (5), pour la réception du tissu (15) à manipuler et/ou à ponctionner, et
- comporte des moyens (7) pour l'intégration d'un vide dans l'espace intérieur (2a),
- dans lequel il est prévu au moins un moyen (12) pour la fixation mécanique et/ou l'immobilisation du tissu (15) sur la dans la région de l'ouverture (2b) de la tête d'aspiration (6),
**caractérisé en ce que**
- l'au moins un moyen (12) pour la fixation mécanique et/ou l'immobilisation du tissu (15) sur la tête d'aspiration (6) est un élément de serrage ou de blocage élastique.

2. Dispositif selon la revendication 1, **caractérisé en ce que** chaque élément de serrage ou de blocage élastique (12) forme un ressort, une langue ou une lèvre élastique (12a), qui s'introduit au moins partiellement dans l'ouverture (2b).

3. Dispositif selon la revendication 1 ou 2, **caractérisé en ce que** l'élément de serrage ou de blocage élastique (12) forme un bord élastique périphérique (12b) pour l'ouverture (2b).

4. Dispositif selon l'une des revendications 1 à 3, **caractérisé en ce que** la section transversale de l'ouverture (12c) délimitée par les éléments de serrage ou de blocage élastiques (12) est inférieure à la section transversale de l'espace intérieur (2a) délimité par l'ouverture (2b).

5. Dispositif selon l'une des revendications 1 à 4, **caractérisé en ce que** les éléments de serrage ou de blocage élastiques (12) sont activement modifiables ou mobiles.

6. Dispositif selon l'une des revendications 1 à 5, **caractérisé en ce que** les éléments de serrage ou de blocage élastiques (12) sont mobiles de façon mécanique, électrique, magnétique, hydraulique ou pneumatique.

7. Dispositif selon l'une des revendications 1 à 6, **caractérisé en ce que** le corps pénétrant (1) est guidé de façon coulissante à l'extérieur de la tête d'aspiration (6).

8. Dispositif selon l'une revendications 1 à 7, **caractérisé en ce que** les moyens (3a) pour le guidage du corps pénétrant (1) sont conçus séparément des moyens (7) pour l'intégration d'un vide dans l'espace intérieur (2a).

9. Revendication selon l'une des revendications 1 à 8, **caractérisé en ce que** dans la tête d'aspiration (6), il est prévu au moins un autre guidage (33) pour un autre corps pénétrant (18).

10. Dispositif selon la revendication 9, **caractérisé en ce que** l'autre guidage (33) est conçu pivotant par rapport à la tête d'aspiration (6).

11. Dispositif selon l'une des revendications 1 à 10, **caractérisé en ce que** la tête d'aspiration (6) comporte au moins un évidement (13), l'évidement (13) étant formé dans une région de bord de la tête d'aspiration (6).

12. Dispositif selon l'une des revendications 1 à 11, **caractérisé en ce qu'**un dispositif de coupe (20) pour le tissu (15) est formé sur la tête d'aspiration (6).
